# EUROPEAN PATENT APPLICATION

(11) **EP 1 222 901 A2**
(43) Date of publication of application: **17.07.2002**
(21) Application number: 02250311.4
(22) Date of filing: 16.01.2002
(51) Int. Cl.: A61F 2/06, C22F 1/00, C23C 8/16

(54) **Medical devices and methods for their manufacture**

(30) Priority: 16.01.2001 US 760595
(71) Applicant: Nitinol Development Corporation, Fremont, California 94539 (US)
(72) Inventor: Pelton, Alan R., Fremont, CA 94536 (US); Trepanier, Christine, Fremont, CA 94538 (US)
(74) Representative: Belcher, Simon James

(57) **Abstract**

A medical device which includes a component formed from an alloy which contains at least about 40% Ni by weight. The alloy in a 10 nm deep surface region of the component contains not more than about 10% Ni by weight. The Ni content in that surface region can be reduced by polishing and oxidizing treatment such as (a) exposure to superheated steam, or (b) immersion in a chemical solution, or (c) an electrochemical treatment, using the device as the anode in a solution bath with a current running therethrough.

## Description

This invention relates to medical devices and to a method of making medical devices. More particularly, this invention relates to stents and a method for making stents. Most particularly, this invention relates to self-expanding Ni-Ti stents and methods of making such stents.

The use of nickel containing alloys in medical devices is well established. Examples of such alloys are nickel-titanium-based alloys, which are used because of their ability to exhibit shape memory properties associated with transformations between the martensitic and austenitic phases. These properties include thermally induced changes in configuration in which an article is first deformed from a heat-stable configuration to a heat-unstable configuration while the alloy is in its martensitic phase. Subsequent exposure to increased temperature results in a change in configuration from the heat-unstable configuration towards the original heat-stable configuration as the alloy reverts from its martensite phase to its austenite phase.

Shape memory alloys can also exhibit enhanced elastic properties compared with materials that do not exhibit martensite-austenite transformations. The nature of the superelastic transformations of shape memory alloys is discussed in "Engineering Aspects of Shape Memory Alloys", T W Duerig *et al*, 370, Butterworth-Heinemann (1990). A principal transformation of shape memory alloys involves an initial increase in strain, approximately linearly with stress. This behaviour is reversible, and corresponds to conventional elastic deformation. Subsequent increases in strain are accompanied by little or no increase in stress, over a limited range of strain to the end of the "loading plateau". The inflection point on a stress v strain graph defines the loading plateau stress. Subsequent increases in strain are accompanied by increases in stress. Upon unloading, there is a decline in stress with reducing strain to the start of the "unloading plateau" evidenced by the existence of an inflection point along which stress changes little with reducing strain. At the end of the unloading plateau, stress reduces, with reducing strain. The inflection point on the stress v graph also defines the unloading plateau stress. Any residual strain after unloading to zero stress is the "permanent set" of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", *supra* at 376.

Many Ni-Ti alloys are considered biocompatible. However, it can be desirable for some applications to use an alloy in which the nickel content is outside the range considered acceptable for *in vivo* use, in particular to achieve a desired physical behaviour in the alloy. One recently employed method to obtain a low-Ni, biocompatible surface is to use electrochemical methods. One particular concept described by this invention is to disclose alternative means to simultaneously decrease Ni content and increase biocompatibility in Ni-Ti alloys.

The present invention provides medical devices whose biocompatibility in relation to nickel content is improved.

Accordingly, in one aspect, the invention provides a medical device which includes a component formed from an alloy which contains at least about 40% Ni, the alloy in a 10 nm deep surface region of the component containing not more than about 10% Ni.

It has been found that the reduction of the nickel content of the alloy in a surface region of the Ni-alloy component can make the device of the invention more easily accepted for *in vivo* use by reducing the risk of toxic or carcinogenic side effects and by preventing its physical degradation. Preferably, the Ni content in the said surface region is reduced to not more than about 5%, more preferably not more than about 3%, especially not more than about 1.5%.

Preferably, the alloy from which the component is formed is a Ni-Ti based alloy, for example a Ni-Ti binary alloy. However, the alloy can contain more than two elements, for example a ternary alloy or a quaternary alloy. Examples of elements that can be included in a Ni-Ti based alloy include Fe, Cu, Co, Zr, Hf, B and Nb.

Preferably, the alloy contains at least about 48% Ni, more preferably at least about 50%. An example of a particularly preferred alloy is a binary alloy containing about 50.8% Ni.

The Ni content in the surface region can be determined by known spectroscopic techniques such as Auger spectroscopy, X-ray Photoelectron Spectroscopy (XPS) or Secondary Ion Mass spectroscopy (SIMS). The content is measured in a 10 nm deep layer over the area in which the component has been treated.

In another aspect, the invention provides a method of making a medical device comprising a component formed from an alloy which contains nickel, which includes the step of exposing the component in a surface region thereof to a treatment which causes the Ni content of the alloy in that region to be reduced compared with that in the remainder of the component.

The Ni content of the alloy in the said surface region can be reduced by specialized oxidizing treatments. Whereas, several investigators have tried to improve corrosion resistance by thermal oxidation heat treatments, there has been little success. Examples of treatments to which the component can be exposed include exposure to a chemical solution, exposure to superheated steam, and an electrochemical treatment. Such treatments can cause alloy elements other than nickel to be oxidized, to form an oxide layer on the component. Simultaneously, these treatments will effectively remove surface Ni atoms, thereby promoting the oxidation of Ti. For example, treatment of a Ni-Ti alloy can result in the formation of a surface layer of TiO₂.

A treatment which involves exposure to superheated steam will preferably involve exposure for at least about 1.5 hours, preferably at least about 3 hours, more preferably at least about 5 hours. Preferably, the steam is heated to at least about 120°C, more preferably at least about 150°C.

In electrochemical treatments of the alloy, the component is preferably included in an electrochemical system as the anode.

In chemical treatments of the alloy, the component is preferably immersed in acidic or basic solutions to modify the surface chemistry.

The Ni alloy component of the device is preferably treated so that it exhibits shape memory properties making it suitable for the particular application for the device. For example, the component can be treated so that it exhibits a thermally induced change in configuration as a result of a change in phase between austenite and martensite phases due to a change in temperature. For many applications, the component will be treated so that it exhibits enhanced elastic properties such as those referred to as "superelastic" properties.

The medical device of the invention can be designed for any of a large number of applications. A particularly preferred device is a stent formed from an alloy, which has been treated so that it exhibits enhanced elastic properties. A stent can be inserted into a lumen while constrained in a transversely compressed configuration, and then allowed to expand so that it contacts the wall of the lumen to support it, and in some applications, also radially forced outwardly. The stent is left *in situ* in the lumen to provide this support to the lumen. The reduction of the risk of complications due to adverse biocompatibility reactions that can be achieved by the present invention gives rise to particular advantages in a device that is used in this way.

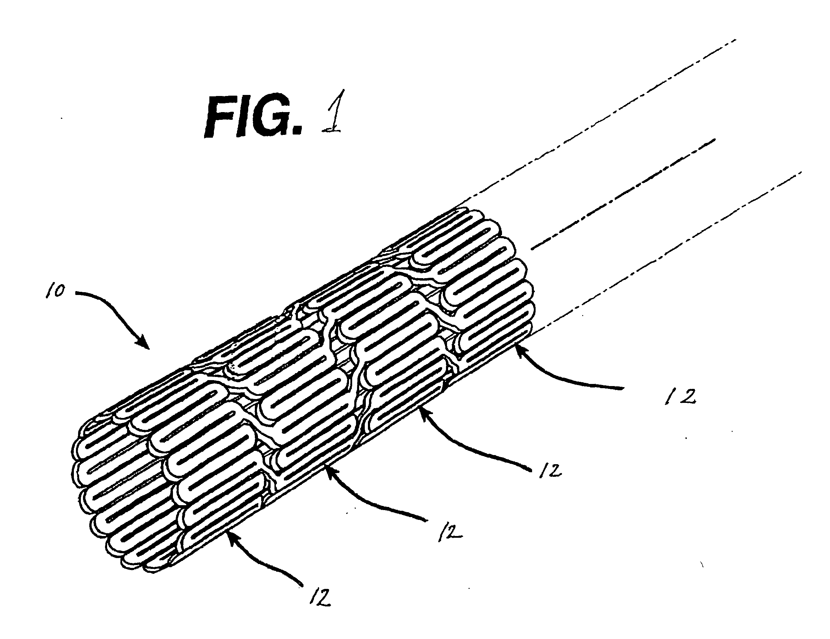

The invention will be better understood by reference to the drawings, in which Figure 1 is a perspective view of a stent made according to the process described herein.

Ni-Ti alloys, containing between 50% and 60% nickel by weight, are currently of great interest to the medical industry due to their superelastic and shape memory properties. These properties provide value for the design of various medical implants, such as stents, suture and bone anchors, archwire, and orthopaedic devices. As is well understood, implants made from these alloys require highly biocompatible surface finishes. Among the requirements of high biocompatibility are low uniform corrosion rates, high resistance to localized corrosion, low toxicity, and low thrombogenicity.

Unalloyed nickel is considered a toxic and carcinogenic substance. While NiTi alloys contain high nickel content, such a characteristic does not result in toxicity *per se*. In order to be toxic, nickel must be released to the environment of the device through corrosion processes. Thrombogenicity is a complicated matter, depending on the design of the device, the environment, surface chemistry and surface roughness.

The process according to the current invention describes a process to produce a very smooth and Ni-free surface on Ni-Ti. "Ni-free" in this case, is defined as less than 5% nickel in the top 10 nm of the material. The process consists of two steps: polishing and oxidizing.

Ideally, the polishing process should be a polishing step that removes the existing oxide layer and leaves a surface finish of RMS 2 or better. One preferred method would be to electropolish in acidic solutions at temperatures below room temperature. These colder temperature processes result in a lower material removal rate. Lower material removal rates are more conducive to polishing complex parts with small cross sectional areas. (Larger material removal rates dictate larger currents. These cannot be uniformly carried through small cross sectional areas without excessive heating.) Polishing may be improved by preceding treatments to remove oxide layers, such as mechanical polishing and/or chemical etching.

The oxidation process can consist of a steam sterilization treatment, or better a surface treatment in a chemical solution, or even better an anodizing process. All these processes remove the Nickel from the surface and leave behind a surface film of Rutile (TiO₂). Immersion of the Ni-Ti component in an acidic or basic chemical solution will selectively remove the existing surface layer and promote the formation of TiO₂. Anodizing appears to significantly reduce the corrosion current in the passive regime and increase the corrosion potential toward more noble values.

The present invention can perhaps best be understood by reference to an example of a stent made according to the disclosed processes.

### EXAMPLE 1

The invention is described by way of a stent is made from a tube of a Ni-Ti binary alloy. (A stent 10 which may be formed according to the described invention is seen in Figure 1.)

The tube from which the stent is formed is made from a Ni-Ti binary alloy which contains 50.8% Ni by weight. The tube has an external diameter of about 3 mm and a wall thickness of about 0.4 mm. Any conventional tube forming techniques, such as drawing, etching, etc, may be used to form the tube.

A pattern is cut into the tube so that it adopts a configuration similar to that described in Figure 1, in which there are slots 12 cut out of the tube. Conventional etching and laser cutting techniques may be used to create slots 12 into the tube, so that it forms stent 10. These slots 12 enable the radial dimension of the tube to be changed by causing the struts 14 to deform relative to one another. (It is also well understood that a segment of wire may be formed into slots 12, so that the effect of the expansion is identical to the slots formed by other processes.) Other suitable configurations of stents will be apparent; for example, stents such as the Palmaz™ stent, Palmaz-Schatz™ stent, Crown™ stent, and Bx Velocity™ stent are all suitable designs of stents for the process described above. Suitable cutting techniques include laser machining (for example using a YAG laser), electric discharge machining, chemical etching and machining.

The stent is treated so that the alloy exhibits superelastic properties by a process that includes a succession of cold working and heat treatment steps (such as those which are discussed in US-5843244). The stent is cold worked by fitting it onto a succession of mandrels of increasing sizes. The stent is heat treated after each cold working step by exposing it to an elevated temperature (that is below the re-crystallization temperature of the alloy) while it is constrained in the configuration resulting from the cold work. A suitable heat treatment temperature is in the range 400°C to 450°C. A succession of cold work and heat treatment steps can be used to impart an appropriate amount of cold work to the stent, which could result in a permanent deformation if carried out in a single step.

After the cold work and heat treatment steps, the stent has superelastic properties so that it can be deformed inwardly towards the configuration as cut, and will then recover elastically towards the configuration from which it was deformed inwardly.

The stent 10 could then be electropolished at temperatures below 20°C using methanol-sulfuric acid solutions. If necessary, the stent 10 may be "primed" for polishing, by using prior treatments to remove oxide layers, such as mechanical methods (for example grit blasting) and/or chemical etching.

The Ni content in the alloy in a surface region of the stent is reduced by an oxidizing treatment involving exposure to superheated steam at 150°C for 12 hours. The stent surface resulting from this treatment contains TiO₂. From Auger spectroscopy, the Ni content in a surface region 10 nm deep has been found to be less than 2% by weight.

Further electromechanical methods of polishing medical devices according to this invention are certainly possible and even likely. For instance, the sulfuric acid bath can be adequately substituted with other acid-based solutions, such as HNO₃, perchloric acid, etc. Basically, the concept applied by this step is to place the medical device into the solution bath, apply a potential, and use the device as an anode in the process. Under proper conditions, electropolishing selectively removes base material, which may contain contaminants, and allows regrowth of the passive oxide on the device surface with a low nickel content contained thereon.

The electromechanical methods described above may further be substituted with purely chemical methods. For instance, one may use one of the following solutions:
Acidic, for example 10% to 50% HNO₃ (preferably 20% to 40% HNO₃; more preferably 30% HNO₃ - water solution);
Neutral, for example saline-based, such as 0.2% to 5% NaCl, preferably 0.5% to 1.5% NaCl, more preferably 0.9% NaCl - water solution;
Basic, for example NaOH solutions.

The purpose of these solutions is to provide a chemical environment to "leach" excess Nickel and other contaminants from the existing oxide surface and allow growth of a more passive (TiO₂) oxide layer. Again, these surfaces possess lower Nickel content than the levels present in typical NiTi medical devices.

In addition, the step may include anodizing. This includes exposure of the medical device to a chemical solution (acidic, neutral or basic) with an appropriately applied potential in order to grow a more stable passive oxide layer. For example, one procedure would be to immerse the NiTi medical device in a saline-based solution, and hold the potential at about 200mV to 1000mV, preferably 300mV to 700mV, more preferably 500mV for 0.1 to 10 hours, preferably 0.2 to 2 hours, more preferably 0.5 to 1 hour.

Of course, it is to be understood that a broad range of equivalent steps are quite possible by which to make stents according to the present invention. For instance, the rates of polishing may be adjusted depending on the desired surface finish. Also, the length of time to heat the stent with superheated steam may be adjusted depending on the size of the stent, or the number of stents heated in a batch. Furthermore, for chemical treatments, the volume of solution and duration of exposure will be adjusted according to the number of stents and the prior processing steps.

## Claims

1. A medical device which includes a component formed from an alloy which contains at least about 40% Ni by weight, the device having a 10 nm deep surface region of containing not more than about 5% Ni by weight, preferably not more than about 3% Ni by weight.

2. A device as claimed in claim 1, in which the alloy has been subjected to polishing and oxidizing treatment on said surface region.

3. A device as claimed in claim 2, in which the said polishing treatment comprises an electrochemical or mechanical treatment.

4. A device as claimed in claim 2, in which the said oxidizing treatment comprises at the steps of exposure to superheated steam, a chemical treatment and an electrochemical treatment.

5. A device as claimed in claim 4, in which the said electrochemical oxidizing treatment comprises anodizing in a acidic, neutral or basic solution.

6. A device as claimed in claim 1, in which the device has been treated so that it exhibits superelastic properties.

7. A device as claimed in claim 1, in which the alloy contains at least about 48% Ni by weight, preferably at least about 50% Ni by weight.

8. A device as claimed in claim 1, in which the alloy is a Ni-Ti based alloy.

9. A device as claimed in claim 1, in the form of a stent.

10. A method of making a medical device comprising a component formed from an alloy which contains nickel, which includes the step of exposing the component in a surface region thereof to a treatment which causes the Ni content of the alloy in that region to be reduced compared with that in the remainder of the component.

11. A method as claimed in claim 10, wherein said device is a stent.

12. A method as claimed in claim 10, in which the component is exposed to superheated steam, preferably for at least about 3 hours, in which the steam is preferably heated to at least about 120°C.

13. A method as claimed in claim 10, in which the said treatment comprises a chemical treatment, in which the device is immersed in an acidic, neutral or basic chemical solution bath, preferably for at least about 0.5 hour.

14. A method as claimed in claim 10, in which the said treatment comprises an electrochemical treatment, in which the device is included in an electrochemical system as an anode in a solution bath with current running therethrough.
